# EUROPEAN PATENT APPLICATION

(11) **EP 2 153 983 A1**
(43) Date of publication of application: **17.02.2010**
(21) Application number: 08740019.8
(22) Date of filing: 08.04.2008
(51) Int. Cl.: B32B 3/30, A61F 13/15, A61F 13/511, B32B 3/24

(54) **LAMINATED BODY OF SHEET-LIKE MEMBER**

(30) Priority: 01.06.2007 JP 2007147476
(71) Applicant: Uni-Charm Corporation, Ehime 7990111 (JP)
(72) Inventor: KURODA, Kenichiro, Kanonji-shi Kagawa 769-1602 (JP); NODA, Yuki, Kanonji-shi Kagawa 769-1602 (JP); NISHIKAWA, Kumiko, Kanonji-shi Kagawa 769-1602 (JP)
(74) Representative: Sperling, Rüdiger
(86) International application number: PCT/JP2008/056916
(87) International publication number: WO 2008/146541

(57) **Abstract**

The size of portions 31p that are included in joining depressions 31 and do not contribute to joining is reduced.

A layered body of sheet-like members includes: a first sheet-like member 11 that includes a plurality of through holes 12 having a same longitudinal direction; a second sheet-like member 21 on which the first sheet-like member 11 is placed; and a plurality of joining depressions 31 that are formed in at least either one of the first sheet-like member 11 and the second sheet-like member 21, and that joins the first sheet-like member 11 and the second sheet-like member 21 to each other, a longitudinal direction of each of the joining depressions 31 slanting with respect to the longitudinal direction of the through holes 12.

## Description

### Technical Field

The present invention relates to a layered body of sheet-like members.

### Background Art

As an absorbent article for absorbing fluid discharged from the human body, such as menstrual blood, for example, a sanitary napkin is known. The absorbent article includes an absorbent body made of a pulverized pulp and the like, and the surface side (the side that is brought into contact with the human skin, the skin side) of the absorbent body is covered with a fluid-permeable sheet (see JP-A-2005-348938, for example).

### Disclosure of Invention

### Problem to be Solved by the Invention

This fluid-permeable sheet is a layered sheet body, for example, having a two-layer structure in which two thermoplastic sheets are superposed. More specifically, the two thermoplastic sheets that are superposed are subjected to embossing, and joined to each other by welding at a plurality of embossed depressions formed by the embossing.

Herein, as a surface side sheet of the layered sheet body, in some cases, a sheet in which a plurality of through holes that penetrate the sheet in the thickness direction are formed in a predetermined area is used. The reason for this is that having the through holes gives the sheet a mesh-like external appearance. Therefore, the sheet appears to absorb menstrual blood well, which increases the user's sense of security.

Moreover, in some cases, it is considered better that the size (the planar size) of the embossed depressions is small. The reason for this is that if the embossed depressions are large, the bulkiness of the layered sheet body is impaired, or gives an uncomfortable feeling.

However, as the size of the embossed depressions is made smaller, a situation is more likely to occur in which an embossed depression that has been formed entirely fits within one of the through holes, or in which most of the embossed depression lies within the through hole. The portion of the embossed depression that has been formed within the through hole in this manner does not contribute to the joining of the sheets. Thus, there is a risk that the joining strength of the sheets will become smaller than the original planned value.

The invention was made in light of conventional problems as described above, and it is an object thereof to provide a layered body of sheet-like members in which it is possible to reduce the size of a portion that is included in a joining depression and that does not contribute to joining when a plurality of joining depressions join a sheet-like member having a plurality of through holes and another sheet-like member.

### Means for Solving the Problem

A primary aspect of the invention for achieving the foregoing object is a layered body of sheet-like members, including:
a first sheet-like member that includes a plurality of through holes having a same longitudinal direction;
a second sheet-like member on which the first sheet-like member is placed; and
a plurality of joining depressions that are formed in at least either one of the first sheet-like member and the second sheet-like member, and that joins the first sheet-like member and the second sheet-like member to each other, a longitudinal direction of each of the joining depressions slanting with respect to the longitudinal direction of the through holes.

Other features of the invention will become clear by reading the description of the present specification with reference to the accompanying drawings.

### Effect of the Invention

According to the invention, there can be provided a layered body of sheet-like members in which a size of a portion that does not contribute to joining of joining depressions can be reduced when a sheet-like member including a plurality of through holes and another sheet-like member are joined with the joining depressions.

### Brief Description of Drawings

[FIG. 1] FIG. 1 is a plan view of a layered body 1 of sheet-like members according to a first embodiment.
[FIG. 2] FIG. 2 is an enlarged perspective view of the layered body 1 of sheet-like members.
[FIG. 3] FIG. 3 is a cross-sectional view taken along line II-II of FIG. 1.
[FIG. 4] FIGS. 4A to 4C are plan views of a through hole 12 and an embossed depression 31.
[FIG. 5] FIG. 5 is an explanatory diagram of a production method of a first sheet-like member 11.
[FIG. 6] FIG. 6 is a perspective view of a second station S2.
[FIG. 7] FIG. 7 is an enlarged perspective view of a belt 73.
[FIG. 8] FIG. 8 is an explanatory diagram of a fiber orientation of the first sheet-like member 11.
[FIG. 9A] FIG. 9A is a plan view of a surface side of an absorbent article 81.
[FIG. 9B] FIG. 9B is a cross-sectional view taken along line B-B of FIG. 9A.
[FIG. 10] FIG. 10 is a plan view of a surface side of an absorbent article 81 in which a layered body 1 of sheet-like members according to a second embodiment is used.
[FIG. 11] FIG. 11A is a plan view that shows a preferred orientation of a heart-shaped embossed depression 31, and FIG. 11B is a plan view that shows an unfavorable orientation.
[FIG. 12] FIG. 12 is a plan view of a surface side of an absorbent article 81 for illustrating other embodiments.

### List of Reference Numerals

1 layered body of sheet-like members,
11 first sheet-like member, 11a surface, 11b back face,
11w fibrous web, 11wf first sheet-like member,
12 through hole, 13 groove section, 14 crest section,
21 second sheet-like member, 21a surface, 21b back face,
31 embossed depression (joining depression), 31a bottom, 31e end section, 31p portion,
31v pointed portion at tip, 31s starting point, 31r embossed depression line,
31w line segment,
33 low-compression depression, 33t groove section,
71 belt conveyor, 73 belt, 74 strip plate,
75 air header, 76 nozzle,
77 suction box, 78a roller,
81 absorbent article, 83 absorbent body, 83e end edge,
87 back face sheet, 89 side sheet,
G: gap, Z: point assumed to face vaginal opening, f: fiber,
S1: first station, S2: second station, S3: third station,
CL31: center line

### Best Mode for Carrying Out the Invention

At least the following matters will be made clear by the explanation in the present specification and the description of the accompanying drawings.

A layered body of sheet-like members, including:
a first sheet-like member that includes a plurality of through holes having a same longitudinal direction;
a second sheet-like member on which the first sheet-like member is placed; and
a plurality of joining depressions that are formed in at least either one of the first sheet-like member and the second sheet-like member, and that joins the first sheet-like member and the second sheet-like member to each other, a longitudinal direction of each of the joining depressions slanting with respect to the longitudinal direction of the through holes.

According to such a layered body of sheet-like members, at the joining depressions, it is possible to reduce the size of portions that do not contribute to the joining between the first sheet-like member and the second sheet-like member. This will be described in detail below. For example, in the case where the joining depressions are substantially the same size as the thorough holes, the joining depression entirely or mostly fits within the through hole when the joining depression is formed overlapping a through hole. In that case, the portion of the joining depression formed within the through hole does not contribute to the joining between the first sheet-like member and the second sheet-like member. Moreover, the size of this portion that does not contribute to the joining increases when the longitudinal direction of the through hole and the longitudinal direction of the joining depression are aligned with each other (when these longitudinal directions are parallel to each other).

In this regard, in the above-described layered body of sheet-like members, the longitudinal direction of each of the joining depressions slants with respect to the longitudinal direction of the through holes. Thus, the joining depressions are unlikely to fit within the through holes, or in other words, portions of the joining depressions are likely to extend out from the through holes. Those portions extending out from the through holes contribute to the joining between the first sheet-like member and the second sheet-like member. Therefore, it is possible to reduce the size of portions that are included in the joining depressions and do not contribute to the joining.

In the layered body of sheet-like members, it is desirable that the longitudinal direction of each of the joining depressions is perpendicular to the longitudinal direction of the through holes.
According to such a layered body of sheet-like members, the longitudinal direction of each of the joining depressions is perpendicular to the longitudinal direction of the through holes. Thus, even when the joining depressions are formed overlapping the through holes, portions of the joining depressions are most likely to extend out from the through holes. Therefore, it is possible to reduce the size of portions that do not contribute to the joining between the first sheet-like member and the second sheet-like member.

In the layered body of sheet-like members, the first sheet-like member may have a plurality of groove sections, a longitudinal direction of each of the groove sections may be the same as the longitudinal direction of the through holes, the groove sections may be provided side by side in a direction perpendicular to the longitudinal direction of the groove sections, and at least one through hole of the plurality of through holes may be provided on each of the groove sections.

In the layered body of sheet-like members, it is desirable that the first sheet-like member and the second sheet-like member are nonwoven fabrics including thermoplastic fiber, that the first sheet-like member and the second sheet-like member are welded to each other at the joining depressions, and that in the first sheet-like member, an amount of fiber oriented in a direction perpendicular to the longitudinal direction of the through holes is smaller than an amount of fiber oriented in the longitudinal direction of the through holes.
According to such a layered body of sheet-like members, the tensile strength in the direction perpendicular to the longitudinal direction of the through holes, which tends to be weak in the first sheet-like member, is effectively reinforced by the joining depressions. Accordingly, the tensile strength in this direction can be increased.

In the layered body of sheet-like members, it is desirable that a size of each of the joining depressions in the direction perpendicular to the longitudinal direction of the through holes is larger than a size of the through holes in the direction perpendicular to the longitudinal direction of the through holes by 0.3 to 5 mm.
According to such a layered body of sheet-like members, even when portions of the joining depressions coincide with the through holes, the joining strength can be maintained while suppressing the uncomfortable feeling due to the high stiffness of the joining depressions. More specifically, when the difference is less than 0.3 mm, in the case where a joining depression coincides with a through hole, the joining depression makes little contribution to the joining. When the difference is more than 5 mm, the joining depressions with high stiffness give an uncomfortable feeling or damage the skin. However, by setting the difference to the above-described range, these problems can be effectively prevented.

### ===Layered Product 1 of Sheet-Like Members according to First Embodiment===

### «Layered Product 1 of Sheet-Like Members»

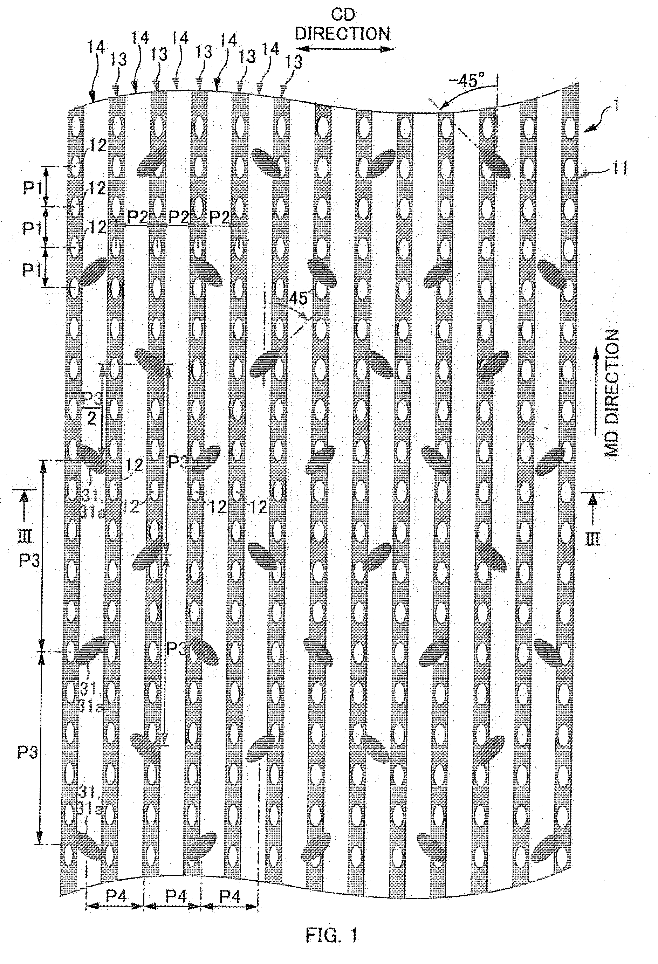

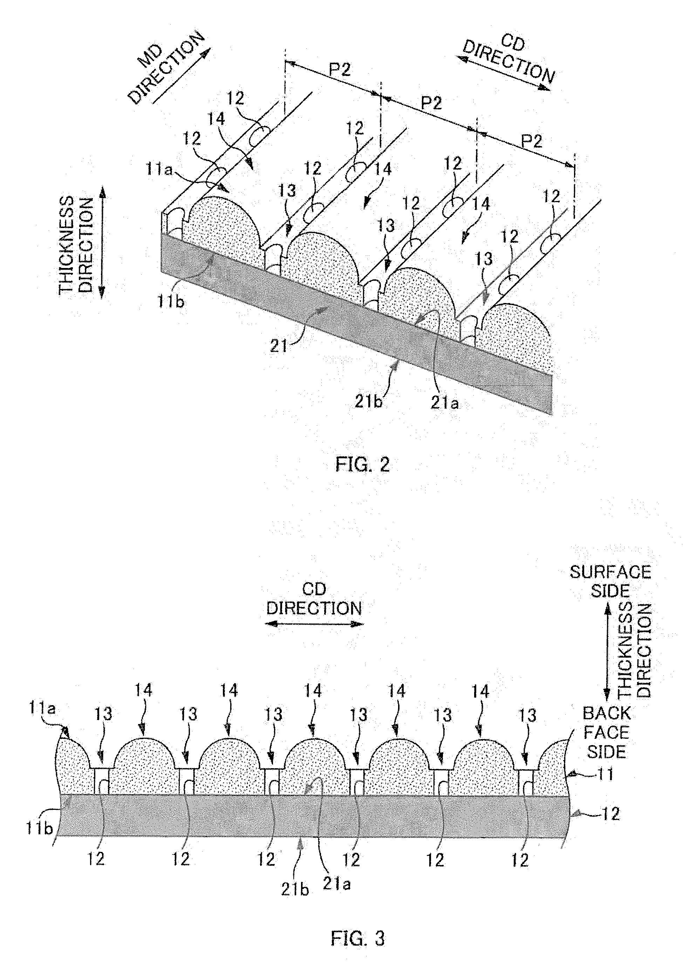

FIG. 1 is a plan view of a layered body 1 of sheet-like members according to a first embodiment, and FIG. 2 is an enlarged perspective view of the layered body 1 of the sheet-like members. FIG. 3 is a cross-sectional view taken along line II-II of FIG. 1. Note that in the following description, as shown in FIG. 1, a direction in which the layered body 1 of the sheet-like members during the production process is continuous is referred to as an MD (Machine Direction) direction, and a direction perpendicular to the MD direction is referred to as a CD (Cross Direction) direction.

As shown in FIGS. 1 to 3, the layered body 1 of the sheet-like members according to the first embodiment has a two-layer structure. That is to say, the layered body 1 of the sheet-like members includes a first sheet-like member 11 having a plurality of through holes 12, the longitudinal direction of each of the through holes 12 being set to the MD direction, and a second sheet-like member 21 on which the first sheet-like member 11 is placed. The two sheet-like members are subjected to embossing in a state in which the two sheet-like members are superposed, and thus, as shown in FIG. 1, a plurality of embossed depressions (corresponding to joining depressions) 31 are formed in the first sheet-like member 11. At these embossed depressions 31, the first sheet-like member 11 and the second sheet-like member 21 are welded and joined together.

Embossing is performed by, for example, passing a layered body in which the first sheet-like member 11 and the second sheet-like member 21 are superposed through a roller gap of a pair of embossing rollers that rotate with heated outer circumferential surfaces facing each other, and pressing the layered body between the embossing rollers in the thickness direction. More specifically, on the outer circumferential surface of one roller of the pair of embossing rollers, a plurality of protrusions having a shape corresponding to the planar shape of the embossed depressions 31 are formed, and the other roller is a smooth roller with a smooth outer circumferential surface. In addition, heads of the protrusions are formed flat. Accordingly, at portions of the layered body that are brought into contact with the protrusions when the layered body is passed through the roller gap of the pair of embossing rollers, depressions having a substantially flat bottom 31a are formed by compression. These depressions are the above-described embossed depressions 31, and at these embossed depressions 31, the first sheet-like member 11 and the second sheet-like member 21 are welded and joined to each other.

Herein, it is considered to be preferable that a size of the embossed depressions 31 (a planar size of the bottom 31a) is reasonably small. This is because when the embossed depressions 31 are large, the bulkiness of the layered body 1 in which the sheet-like members are joined together is impaired, or gives an uncomfortable feeling.

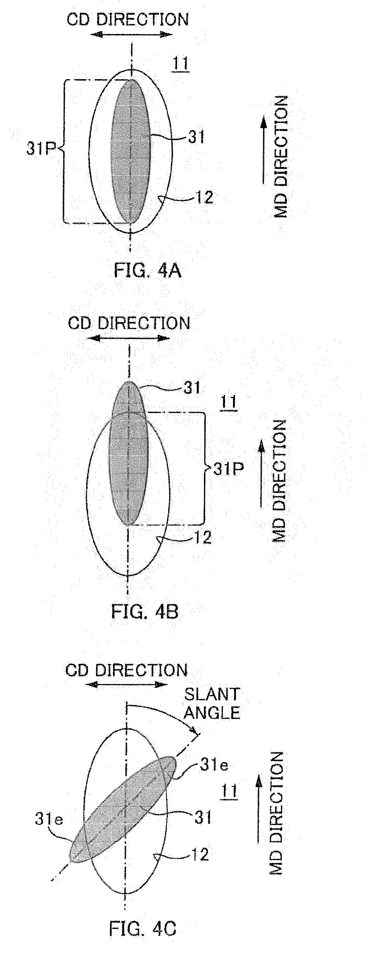

However, as the size of the embossed depressions 31 is made smaller, a situation is more likely to occurs in which, as shown in a plan view of FIG. 4A, an embossed depression 31 is formed to entirely fit within a through hole 12 of the first sheet-like member 11, or in which, as shown in FIG. 4B, most of the embossed depression 31 is formed within the through hole 12. The portion 31p of the embossed depression 31 that is formed within the through hole 12 does not contribute to the joining between the first sheet-like member 11 and the second sheet-like member 21. When this portion 31p of the embossed depression 31 is large, there is a risk that the joining strength between the first sheet-like member 11 and the second sheet-like member 21 would become smaller than the original planned value.

In the first embodiment, a size of the portion 31p that is included in the embossed depression 31 and does not contribute to the joining is reduced by devising a shape of the embossed depression 31. Details will be described below. First, from the standpoint of the shape of the embossed depression 31, the above-described situation in which the embossed depression 31 entirely or mostly fits within the through hole 12 is likely to occur in the case where, as shown in FIGS. 4A and 4B, the longitudinal direction of the through hole 12 and a longitudinal direction of the embossed depression 31 are aligned with each other (i.e., in the case where the longitudinal directions are parallel to each other). On the other hand, this situation is unlikely to occur in the case where, as shown in FIG. 4C, the longitudinal directions are not aligned with each other. More specifically, in the latter case, even when the embossed depression 31 is formed overlapping the through hole 12, an end section 31e in the longitudinal direction of the embossed depression 31 lies greatly outside the through hole 12.

For this reason, in the layered body 1 of sheet-like members according to the first embodiment, as shown in FIG. 1, the longitudinal direction of each of the embossed depressions 31 slants with respect to the MD direction, which is the longitudinal direction of the through holes 12, thereby reducing the size of the portions 31p that are included in the embossed depressions 31 and do not contribute to the joining.

Hereinafter, the elements of the layered body 1 of sheet-like members according to the first embodiment will be described.

### <<<First Sheet-Like Member 11>>>

The base material of the first sheet-like member 11 is, for example, a nonwoven fabric containing thermoplastic resin fiber and having a generally uniform thickness. Examples of the thermoplastic resin fiber include single fiber made of polyethylene (hereinafter referred to as PE), polypropylene (hereinafter referred to as PP), polyethylene terephthalate (hereinafter referred to as PET), or the like, fibers produced by polymerizing PP and PE, or composite fibers made of PP and PE and having a core-sheath structure. Note that the nonwoven fabric may contain a fiber other than thermoplastic resin fiber. For example, the nonwoven fabric may contain natural fiber such as cellulose.

In this first sheet-like member 11, as shown in FIG. 1, the through holes 12 that penetrate the first sheet-like member 11 in the thickness direction are formed over the entire surface of the first sheet-like member 11 in a substantially lattice-like pattern at a formation pitch P1 in the MD direction and a formation pitch P2 in the CD direction. The through holes 12, for example, have elliptical shapes of equal size when viewed from above, and for every through hole 12, the major axis (corresponding to the longitudinal direction) extends in the MD direction, and the minor axis extends in the CD direction.

Moreover, as shown in FIGS. 3 and 2, although the back face 11b of the first sheet-like member 11 is generally flat, on the surface 11a of the first sheet-like member 11, rectilinear groove sections 13 is formed in the MD direction at positions in the CD direction where the through holes 12 are formed. More specifically, on the surface 11a, the groove sections 13 whose longitudinal direction is the MD direction are formed at the formation pitch P2 in the CD direction. Thus, the cross-section of the first sheet-like member 11 taken in the CD direction is substantially corrugated on the surface 11a side. Incidentally, crest sections 14 between adjacent groove sections 13 in the CD direction are formed, as described below, in such a manner as fibers that were originally located at the positions of the groove sections 13 are blown toward the crest sections 14 and piled thereat. Thus, the basis weight (g/m²) in the crest sections 14 is larger than that in the groove sections 13.

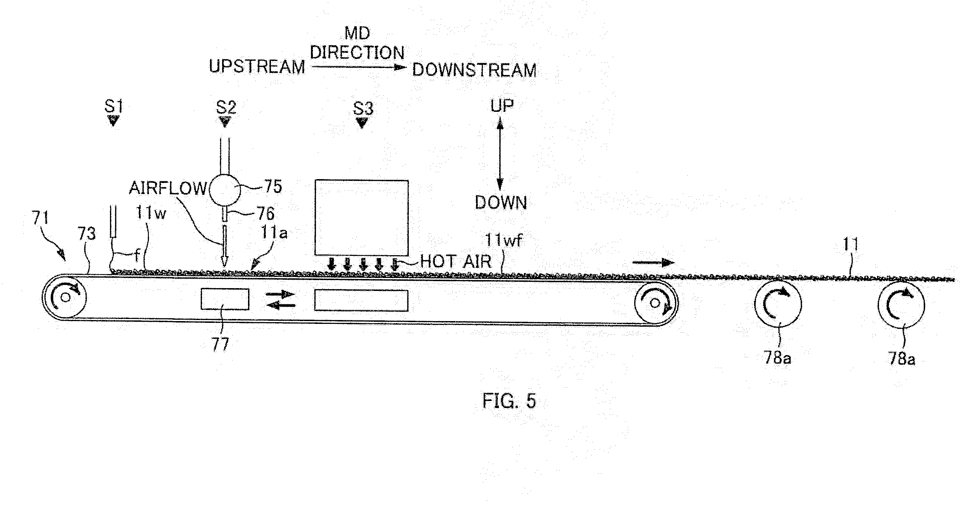

FIG. 5 is an explanatory diagram of a production method of such a first sheet-like member 11. The first sheet-like member 11 is produced in the following manner using, for example, a belt conveyer 71 having a belt 73 moving in the MD direction at a predetermined moving speed.

First, at the furthest upstream first station S1, fibers f that have been spun are deposited on the belt 73 that moves in the MD direction, and thus a fibrous web 11w continuous in the MD direction is formed.

Then, at a second station S2 downstream from the first station S1, an airflow processing is performed. More specifically, a flow of air is blown from an air header 75 against the fibrous web 11w that is placed on the belt 73 and moves downstream in the MD direction together with the belt 73. As a result, the above-described groove sections 13 and through holes 12 are formed on the surface 11a of the fibrous web 11w.

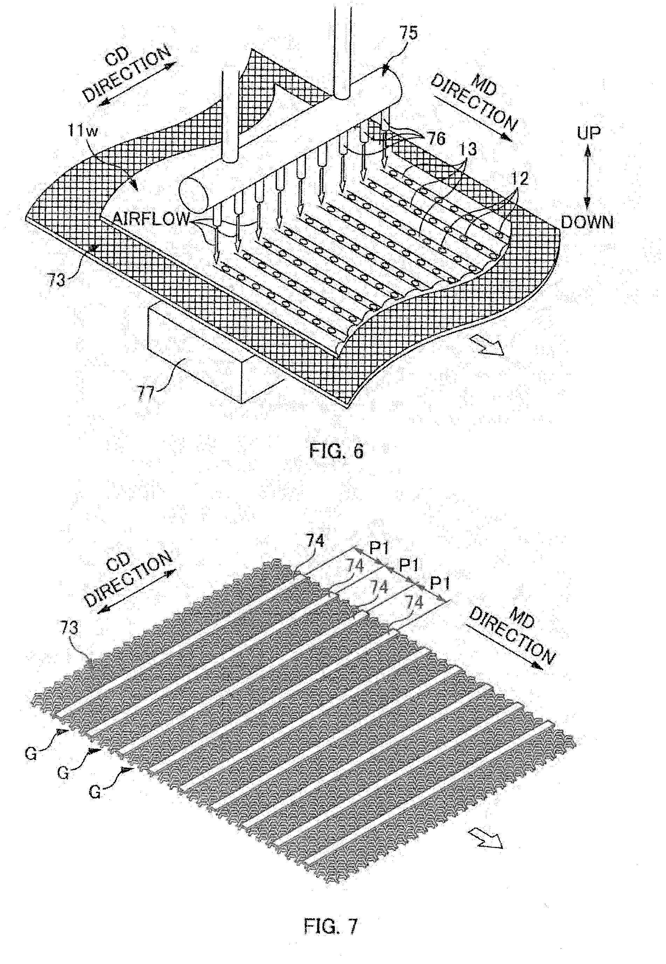

FIGS. 6 and 7 are diagrams for illustrating details of the process of formation of the groove sections 13 and the through holes 12 by the airflow processing. FIG. 6 shows a perspective view of the second station S2, and FIG. 7 shows an enlarged perspective view of the belt 73.

As shown in FIG. 6, on a face that is included the air header 75 and faces the belt 73, nozzles 76 are formed at the formation pitch P2 in the CD direction. In addition, the belt 73 is, for example, a net-like body through which air can pass in the thickness direction (up-and-down direction in FIG. 6), and furthermore, a suction box 77 for sucking the airflow is provided under the belt 73. Accordingly, the airflow ejected from the nozzles 76 of the air header 75 passes through the fibrous web 11w and the belt 73 in the thickness direction, and is then sucked into the suction box 77. At that time, the airflow blows apart fibers located at positions exposed to the airflow and moves the fibers in the CD direction. In this manner, the groove sections 13 are formed at the positions exposed to the airflow. In short, the groove sections 13 extending along the MD direction are formed in the fibrous web 11w at the formation pitch P2 in the CD direction.

Note that, herein, as shown in FIG. 7, air-impermeable, rectangular strip plates 74 extending in the CD direction are disposed on a surface of the net-like belt 73 at the formation pitch P1 in the MD direction. Moreover, strip gaps G are formed between strip plates 74 that are adjacent to each other in the MD direction. For this reason, fibers located in the gaps G are acted on only by a force that blows those fibers apart in the CD direction when the airflow passes in the thickness direction, and thus only the groove sections 13 are formed. However, fibers located at positions on the strip plates 74 are further acted on by, in addition to the aforementioned force, airflow that cannot pass through the strip plates 74 and moves along the surfaces of the strip plates 74, so as to move the fibers in the CD direction. As a result, the through holes 12 having substantially perfect circular shapes are formed at these positions.

After the groove sections 13 and the through holes 12 are formed in the fibrous web 11w in this manner, the fibrous web 11w is moved to a third station S3 downstream from the second station S2, as shown in FIG. 5. While the fibrous web 11w passes through the third station S3, the fibrous web 11w is heated with hot air or the like by a method such as an air-through method. As a result, the entangled fibers are fused together, and the first sheet-like member 11wf is almost completed.

Then, the first sheet-like member 11wf is moved further downstream in the form of a continuous sheet. During the movement, the first sheet-like member 11wf is extended across a plurality of rollers 78a, and in this state, the first sheet-like member 11wf is carried while being pulled downstream in the MD direction. Accordingly, due to tension that acts on the first sheet-like member 11wf in the MD direction, the shapes of the through holes 12 are changed from the substantially perfect circles to ellipses with the major axes extending in the MD direction, and thus the first sheet-like member 11 is completed.

### «Second Sheet-Like Member 21»

The base material of the second sheet-like member 21 also is a nonwoven fabric containing thermoplastic resin fiber, or in some cases natural fiber such as cellulose, and having a generally uniform thickness. As the thermoplastic resin fiber, those described above as examples regarding the first sheet-like member 11 are used.
However, as shown in FIGS. 2 and 3, the groove sections 13 and the through holes 12 as in the case of the above-described first sheet-like member 11 are not formed in the second sheet-like member 21, and both a surface 21a and a back face 21b are substantially flat. Therefore, as the production method of the second sheet-like member 21, a usual nonwoven fabric production method is used. More specifically, as the method for forming the fibrous web, a dry process (e.g., air laying), a wet process, or a process in which fibers are spun and then directly dispersed into a web (e.g., spunbonding or meltblowing) can be given as an example, and as the method for bonding the fibers, thermal bonding, needle punching, chemical bonding, or spunlacing can be given as an example.

### «Embossed Depressions 31»

As described above, the embossed depressions 31 are joining sections that join the first sheet-like member 11 and the second sheet-like member 21 to each other. As shown in FIG. 1, a plurality of embossed depressions 31 with elliptical bottoms 31a when viewed from above are formed on the surface 11a of the first sheet-like member 11 in a predetermined formation pattern.

The formation pattern shown as an example in the drawings is what is called a staggered pattern. More specifically, at a formation pitch P4 in the CD direction, a plurality of embossed depression lines are formed in each of which a plurality of embossed depressions 31 are arranged in a straight line at a formation pitch P3 in the MD direction, and adjacent embossed depression lines in the CD direction are staggered by half the formation pitch P3 in the MD direction.

Herein, in this first embodiment, every embossed depression 31 is formed with the major axis direction, which is the longitudinal direction of the embossed depression 31, slanting at a slant angle of 45° or 135° (-45°) from the MD direction, which is the major axis direction of the through holes 12. The purpose of this is that even when an embossed depression 31 and a through hole 12 are formed at overlapping positions when viewed from above, a portion of the embossed depression 31 that fits within the through hole 12 is minimized so that the embossed depression 31 lies greatly outside the through hole 12.

Accordingly, the slant angle is not limited to 45° or 135° (-45°), and may be set to any angle as long as the MD direction and the major axis direction are not parallel to each other. However, in order to increase the portion of the embossed depression 31 lying outside the through hole 12 without changing the size (the planar size) of the embossed depression 31, it is most effective to make the major axis direction of the embossed depression 31 perpendicular to the MD direction. Therefore, the slant angle may be set to preferably 10° to 170°, more preferably 30° to 150°, even more preferably 45° to 135°, and most preferably 90°.

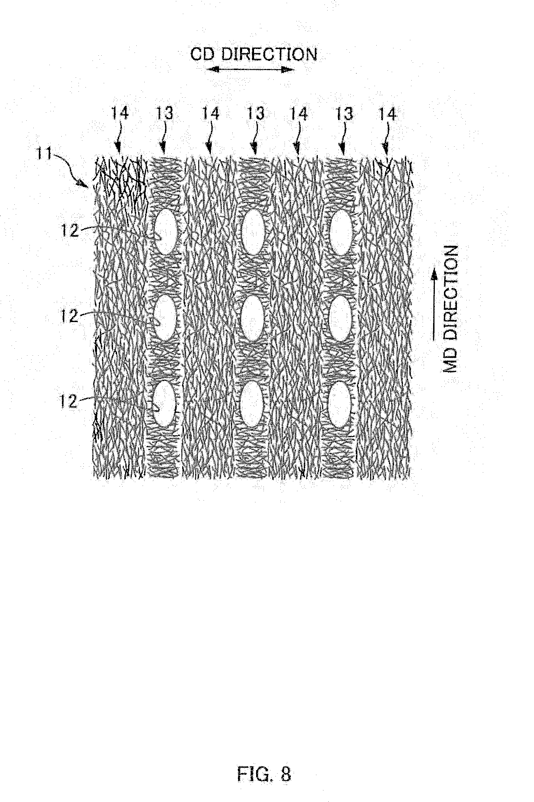

Incidentally, when the major axis direction of each of the embossed depressions 31 slants with respect to the MD direction, it is also possible to increase the tensile strength in the CD direction, which tends to be weak due to the properties of the first sheet-like member 11. In other words, the first sheet-like member 11 has a property that the macroscopic fiber orientation is in the MD direction rather than in the CD direction. Specifically, as schematically shown in a plan view of FIG. 8, an amount of fibers oriented in the MD direction (fibers oriented in any direction within the range of -45° to +45° relative to the MD direction) is larger than an amount of fibers oriented in the CD direction (fibers oriented in any direction within the range of -45° to +45° relative to the CD direction). This is due to the above-described production method. In other words, this is because the fibrous web 11w, which is made into the first sheet-like member 11, is generated by depositing fibers that have been spun on the belt 73 while moving the belt 73 in the MD direction as shown in FIG. 5.

In the case of the first sheet-like member 11 having this property that the fiber orientation is in the MD direction, the fibers tend to be separated in the CD direction, so that the tensile strength in the CD direction is weak. However, herein, in the first sheet-like member 11, the embossed depressions 31 are formed with the major axes slanting with respect to the MD direction. Accordingly, lengths of the embossed depressions 31 in the CD direction are increased by the slant angle. In other words, the embossed depressions 31 are formed straddling many fibers arranged side by side in the CD direction, and thus it is possible to fix more fibers in such a manner as the fibers are not separated. As a result, the tensile strength in the CD direction is increased.

Moreover, as shown in FIG. 1, in some embossed depressions 31, a part or most of an embossed depression 31 is formed in a groove section 13. With these embossed depressions 31, it is possible to increase, by the embossed depressions 31, the tensile strength of the groove sections 13, which tends to be weak because, as described above, the basis weight (g/m²) in the groove sections 13 is smaller than that in the crest sections 14.

### ===Application Example of Layered Product 1 of Sheet-Like Members according to First Embodiment===

In this application example, the layered body 1 of sheet-like members according to the first embodiment is used in a sanitary napkin serving as an absorbent article 81. In the following description, a side that is brought into contact with the human body is referred to as a surface side, and a side that is brought into contact with an undergarment is referred to as a back face side.

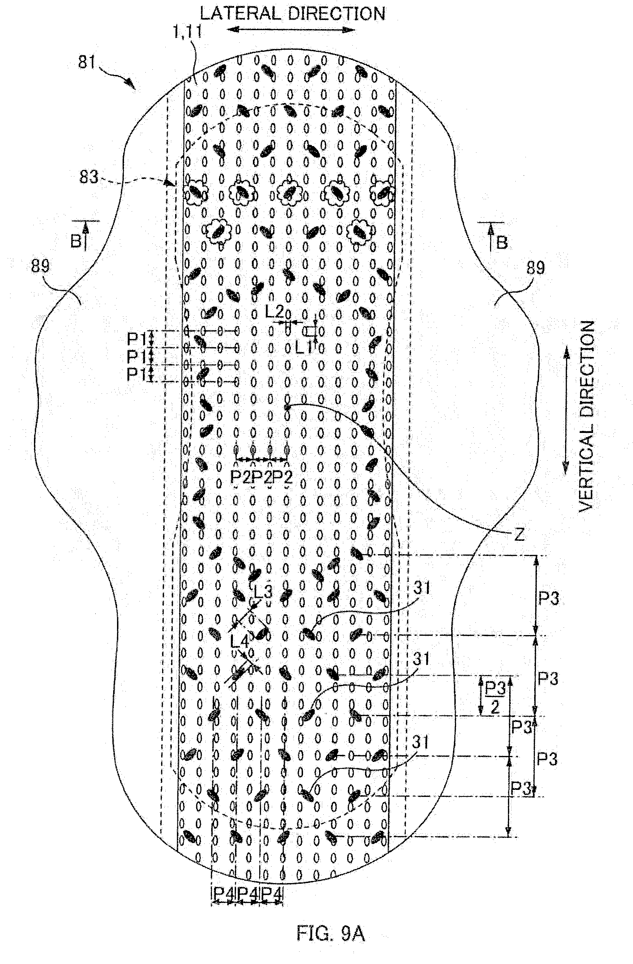

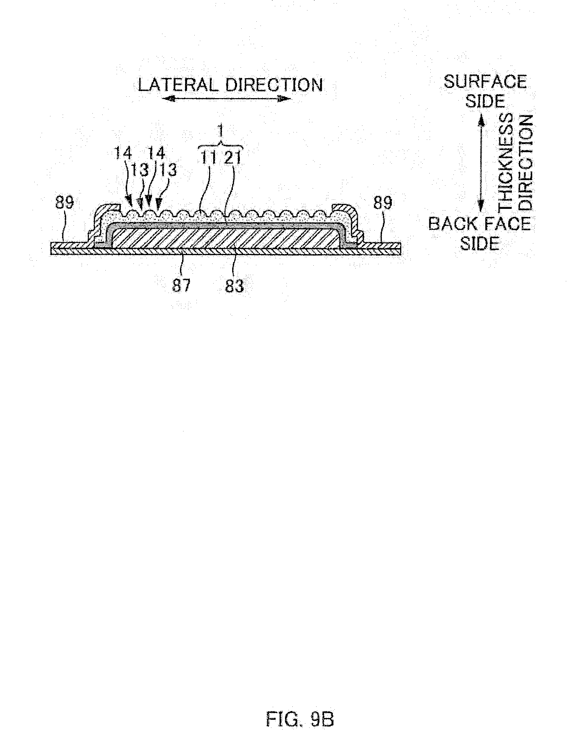

FIG. 9A is a plan view of the surface side of the absorbent article 81. FIG. 9B is a cross-sectional view taken along line B-B of FIG. 9A. As shown in FIG. 9A, the absorbent article 81 is generally elongated in a predetermined direction. In the following description, this predetermined direction is referred to as a vertical direction, and a direction perpendicular to the vertical direction is referred to as a lateral direction. Note that the groove sections 13 are omitted from FIG. 9A so that FIG. 9A is not complicated.

As shown in FIGS. 9A and 9B, the absorbent article 81 includes a substantially rectangular, flat absorbent body 83 that is formed by wrapping a pulverized pulp mixed with a superabsorbent polymer in a fluid-permeable sheet (not shown) such as tissue paper, a fluid-permeable surface sheet member 1 that is provided to cover a surface side of the absorbent body 83, a fluid-impermeable back face sheet 87 that is provided to cover a back face side of the absorbent body 83, and a pair of side sheets 89 that is provided to cover, from the surface side, opposite end sections in the lateral direction of the absorbent body 83 in order to prevent leakage of fluid from opposite end sections in the lateral direction of the absorbent article 81.

As shown in FIG. 9A, the surface sheet member 1 is a fluid-permeable sheet having a substantially rectangular shape slightly larger than the shape of the absorbent body 83 when viewed from above. The above-described layered body 1 of sheet-like members according to the first embodiment is used as this surface sheet member 1. Specifically, as shown in FIG. 9A, the MD direction of the layered body 1 is set to the vertical direction, and as shown in FIG. 9B, the layered body 1 is provided to cover the surface side of the absorbent body 83 with the first sheet-like member 11 on the surface side and the second sheet-like member 21 on the back face side. It is advantageous that the fiber density of the second sheet-like member 21 is higher than that of the first sheet-like member 11. In this case, the surface sheet member 1 can quickly transfer fluid such as menstrual blood discharged from the human body to the absorbent body 83.

Herein, it is desirable that both the formation pitch P1 in the MD direction and the formation pitch P2 in the CD direction of the through holes 12 in the first sheet-like member 11 of the surface sheet member 1 are within the range of 1.5 to 20 mm. The reason for this is as follows. When the formation pitches are smaller than 1.5 mm, an area on the surface of the first sheet-like member 11 occupied by the through holes 12 becomes excessively large, so that the ability to conceal the fluid that has been absorbed by the absorbent article 81 deteriorates. On the other hand, when the formation pitches are larger than 20 mm, the fluid permeability deteriorates. Therefore, in this application example, both of the formation pitches P1 and P2 are 5.3 mm. Moreover, it is desirable that both of the length L1 in the major axis direction and the length L2 in the minor axis direction of the through holes 12 are within the range of 1 to 7 mm. The reason for this is as follows. When the lengths are smaller than 1 mm, the fluid permeability deteriorates. On the other hand, when the lengths are larger than 7 mm, the through holes 12 become excessively large, so that the ability to conceal the fluid that has been absorbed by the absorbent article 81 deteriorates. Therefore, in this application example, the length L1 in the major axis direction and the length L2 in the minor axis direction are 2.8 mm and 1.25 mm, respectively.

Moreover, it is desirable that both of the formation pitch P3 in the MD direction and the formation pitch P4 in the CD direction of the embossed depressions 31 are within the range of 3 to 30 mm. The reason for this is as follows. When the formation pitches are smaller than 3 mm, an area on the surface of the first sheet-like member 11 occupied by the embossed depressions 31 becomes excessively large, so that, for example, gives an uncomfortable feeling. On the other hand, when the formation pitches are larger than 30 mm, it is impossible to secure the joining strength necessary to join the first sheet-like member 11 and the second sheet-like member 21 to each other. Therefore, in this application example, the formation pitch P3 and the formation pitch P4 are 24 mm and 7.5 mm, respectively.

Note that since the lower limit of the formation pitches P3 and P4 is 3 mm, the following advantages also can be obtained. The first sheet-like member 11 of the surface sheet member 1 is produced by the airflow processing shown in FIG. 6, and hence made into a relatively bulky sheet. As a result, it is possible to space the absorbent body 83 from the wearer's skin. Accordingly, the fluid that has been once absorbed by the absorbent body 83 via the surface sheet member 1 can be restrained from returning back to the surface of the surface sheet member 1. In other words, this surface sheet member 1 can reduce the occurrence of situations in which the fluid that has returned back gives a wet feeling on the wearer's skin or makes the wearer's skin dirty. However, when the formation pitches P3 and P4 of the embossed depressions 31 are small, the fibers of the first sheet-like member 11 are flattened by the embossed depressions 31. Thus, the bulkiness is impaired, and the above-described effects cannot be obtained. However, when the lower limit of the formation pitches P3 and P4 is 3 mm as described above, the impairment of the bulkiness can be effectively suppressed, and the above-described features can be achieved.

Furthermore, when the formation pitches P3 and P4 are small, in the case where fibers around the through holes 12 are flattened into the through holes 12 due to the formation of the embossed depressions 31, there is a risk that the fluid that has been once absorbed by the absorbent body 83 will move on the flattened fibers and return back to the surface of the surface sheet member 1. This problem also can be effectively prevented as long as the lower limit of the formation pitches P3 and P4 is 3 mm.

Moreover, it is desirable that both the length L3 in the major axis direction and the length L4 in the minor axis direction of the embossed depressions 31 are within the range of 1.5 to 10 mm. The reason for this is as follows. When the lengths are smaller than 1.5 mm, it is impossible to secure sufficient joining strength between the first sheet-like member 11 and the second sheet-like member 21. On the other hand, when the lengths are larger than 10 mm, the stiffness becomes excessively high, so that, for example, the feel becomes uncomfortable. Therefore, in this application example, the length L3 in the major axis direction and the length L4 in the minor axis direction are 4.0 mm and 1.5 mm, respectively.

Moreover, it is desirable that the size in the CD direction of the embossed depressions 31 is larger than the size in the CD direction of the through holes 12 by 0.3 to 5 mm. The reason for this is as follows. When the difference is less than 0.3 mm, in the case where an embossed depression 31 coincides with a through hole 12, the embossed depression 31 makes little contribution to the joining. In addition, when the difference is more than 5 mm, the embossed depressions 31 with high stiffness are likely to give an uncomfortable feeling to the wearer or to damage the wearer's skin. However, by setting the difference to the above-described range, these problems can be effectively prevented.

In this application example, the embossed depressions 31 are not formed in a substantially rectangular predetermined area whose center is at a point Z that is assumed to face the vaginal opening in the vertical direction and in the lateral direction. This is because the predetermined area is a portion that is brought into the closest contact with the human body and a high bulkiness and an excellent touch are required. Moreover, additional embossed depressions 31 are formed at formation pitches other than the formation pitches P1 and P2 in such a manner as those embossed depressions 31 surround the outline of the predetermined area. The reason for this is that since the predetermined area is located at the position of the crotch, a large sandwiching force from the crotch acts in the lateral direction, and thus there is a risk that the joining at those embossed depressions 31 will separate. In other words, by forming many embossed depressions 31, the magnitude of the sandwiching force distributed to each of the embossed depressions 31 is decreased.

Moreover, in this application example, some embossed depressions 31 are formed partly overlapping the through holes 12. More specifically, some embossed depressions 31 are formed straddling both the inner side and the outer side of the through holes 12 (see the embossed depressions 31 surrounded by the circles adorned with petals in FIG. 9A, for example). With this configuration, fluid discharged from the human body onto the surface sheet member 1 can be quickly guided to the absorbent body 83 via the through holes 12. Specifically, in portions where the embossed depressions 31 are formed, the fiber density is high due to the compressive deformation caused by embossing, so that a permeability based on the capillarity of the fibers also is strong. Accordingly, when the embossed depressions 31 straddle the peripheries of the through holes 12 as described above, fluid that stays around the through holes 12 is guided by the high permeability of the embossed depressions 31 into the through holes 12 via the embossed depressions 31, and quickly absorbed by the absorbent body 83 via the through holes 12.

### ===Layered Product 1 of Sheet-Like Members according to Second Embodiment and Application Example Thereof===

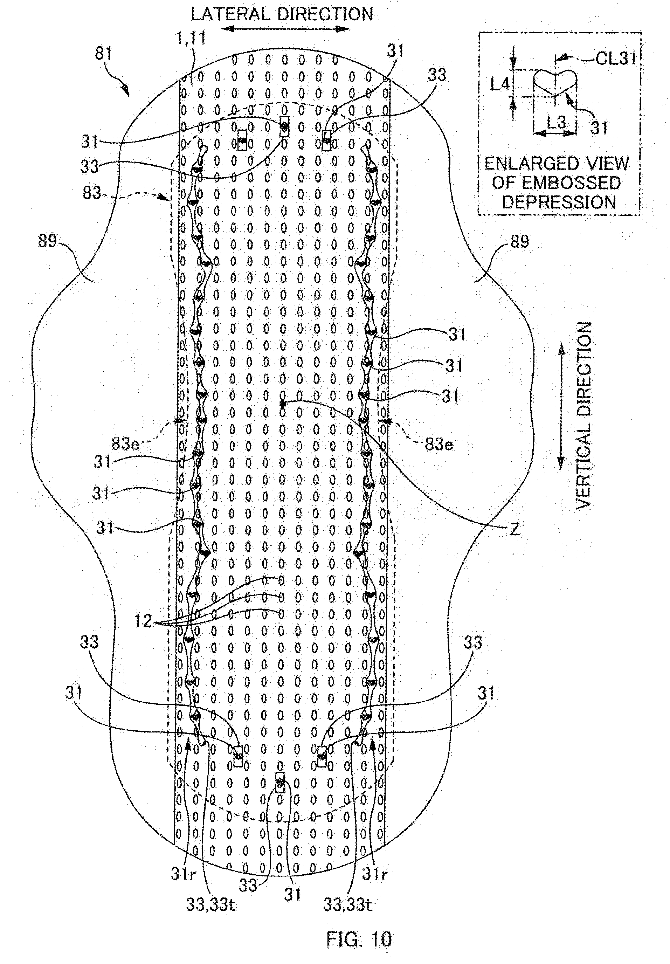

FIG. 10 is a plan view of a surface side of an absorbent article 81 in which a layered body 1 of sheet-like members according to a second embodiment is used. The layered body 1 of sheet-like members is used as the surface sheet member 1 in a similar manner as the first embodiment.

Herein, the second embodiment is different from the first embodiment in the following points. First, the embossed depressions 31 are heart-shaped when viewed from above. Second, the formation pattern of the embossed depressions 31 is not the staggered pattern. Third, a low-compression depression 33 in which the compression amount is small is formed continuously in the front and rear of each of the embossed depressions 31 in the vertical direction. The three points of difference will be described below.

First, regarding the first point of difference, as shown in FIG. 10, embossed depressions 31 that are heart-shaped when viewed from above are formed in the layered body 1 of sheet-like members according to the second embodiment. Each of the heart-shaped embossed depressions 31 are oriented so that their shape is line symmetric. More specifically, a straight line CL31 connecting the tips of a V-shaped portion and a U-shaped portion of the heart shape is the axis of symmetry. Herein, the longer of two directions, namely, a direction of this straight line CL31 and a direction perpendicular to this straight line CL31, is defined as the longitudinal direction. In the example shown in the drawings, the direction perpendicular to the straight line CL31 is longer than the direction of the straight line CL31, which is line symmetric, so that the direction perpendicular to the straight line CL31 is the longitudinal direction, and the direction of the straight line CL31 is a short length direction.

In the second embodiment, the longitudinal direction of each of the embossed depressions 31 is substantially perpendicular to the MD direction (the vertical direction) as shown in FIG. 10. Accordingly, the size of portions extending out from the through holes 12 of the first sheet-like member 11 can be effectively increased.

For the same reason as described in the first embodiment, it is desirable that both of the length L3 in the longitudinal direction and the length L4 in the short length direction of the embossed depressions 31 also are within the range of 1.5 mm to 10 mm. Therefore, in this application example, the length L3 in the longitudinal direction and the length L4 in the short length direction are 3 mm and 1.73 mm, respectively.

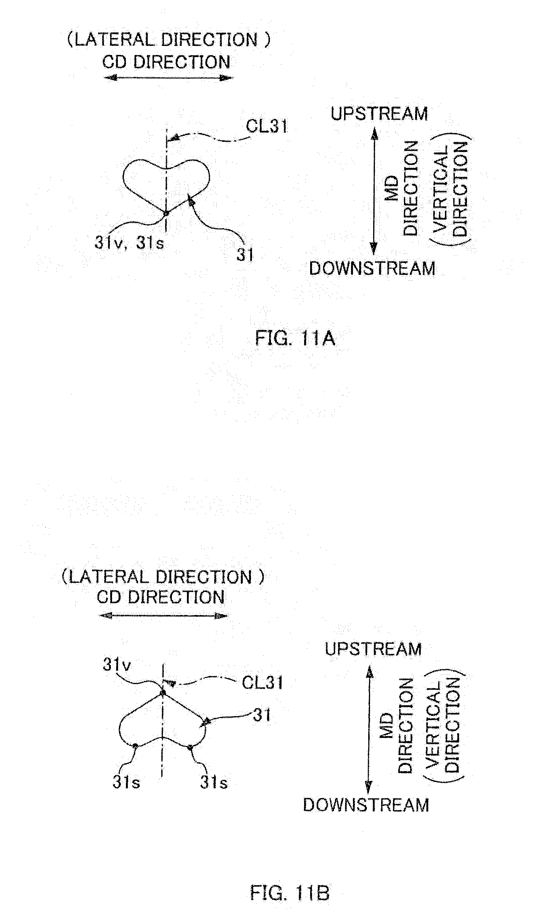

Herein, preferably, it is desirable that as shown in FIG. 11A, a pointed portion 31v at the tip of the V-shaped portion of the heart shape faces the downstream side in the MD direction during the production process (the side toward which the sheet-like members 11 and 21 are advanced during the production process). In other words, when upstream and downstream in the MD direction are in a state shown in FIG. 11A, it is desirable that the pointed portion 31v at the tip is positioned downstream in the MD direction from the other portions of the heart shape as shown in FIG. 11A.

The reason for this is that if the pointed portion 31v at the tip faces the upstream side as shown in FIG. 11B, the sheet-like member 11 is likely to tear when a layered body in which the sheet-like members 11 and 21 are superposed passes through the roller gap of the pair of embossing rollers and processed to form the embossed depressions 31. More specifically, in this case, the processing for forming an embossed depression 31 starts at two points 31s in the CD direction, and there is a risk that a portion between the starting points 31s will be pulled and torn when these starting points 31s are processed. In contrast, if the pointed portion 31v at the tip faces the downstream side as shown in FIG. 11A, the processing starts at a single point 31s, so that a portion to be pulled is unlikely to be generated. As a result, the frequency of tearing also is decreased.

Next, the second point of difference will be described. As shown in FIG. 10, the formation pattern of the embossed depressions 31 in the second embodiment has a basic form in which a plurality of embossed depressions 31 aligned in a substantially vertical direction are each disposed along each end edge 83e in the lateral direction of the absorbent body 83. More specifically, a pair of embossed depression lines 31r in each of which a plurality of embossed depressions 31 are aligned in the substantially vertical direction are formed in such a manner that the embossed depression lines 31r are disposed side by side in the lateral direction with a distance provided between each other. Moreover, in a portion between the pair of embossed depression lines 31r, in opposite end sections in the vertical direction, a plurality of (e.g., three) embossed depressions 31 arranged in the same line in the lateral direction are formed. When these embossed depressions 31 are combined with the above-described pair of embossed depression lines 31r, the embossed depressions 31 as a whole are arranged in the form of a frame along the periphery of an external outline of the absorbent body 83.

Finally, the third point of difference will be described. As shown in FIG. 10, in portions adjacent to each embossed depression 31 on both sides in the vertical direction, the low-compression depression 33, in which the first sheet-like member 11 is deformed by compression in the thickness direction with a compression amount smaller than that in the embossed depression 31, is formed contiguously to the embossed depression 31. For example, as shown in FIG. 10, for each of the three embossed depressions 31 formed in the same line in the lateral direction between the pair of embossed depression lines 31r, the low-compression depression 33 with a depth shallower than that of the embossed depression 31 is formed in the portions adjacent to the embossed depression 31 on both sides in the vertical direction.

This low-compression depression 33 also is for preventing tearing during the processing for forming the embossed depression 31. More specifically, when a large amount of compression is abruptly applied during the processing for forming the embossed depression 31, there is a risk that the sheet-like member 11 cannot follow the abrupt compressive deformation and will tear. Therefore, before and after the processing for forming the embossed depression 31, portions in the front and rear of the embossed depression 31 are preliminarily compressed with a compression amount smaller than that in the embossed depression 31. Thus, the compression deformation is made gradual, and the tearing is prevented.

Regarding the embossed depressions 31 constituting each of the embossed depression lines 31r, since a plurality of embossed depressions 31 are positioned in a line in front of and behind one another in the vertical direction as shown in FIG. 10, low-compression depressions 33 of adjacent embossed depressions 31 in the vertical direction are contiguous. Accordingly, the external appearance of these embossed depressions 31 is a groove section 33t that is formed along the embossed depression line 31r and that contains the embossed depression line 31r therein.

Moreover, the width of this groove section 33t is gently narrowed in such a manner as the width is minimized at middle portions between adjacent embossed depressions 31 in the vertical direction. Since the width is narrowed in this manner, tearing in the highly-compressed embossed depressions 31 is effectively avoided while suppressing the increase in the stiffness caused by the low-compression depressions 33 serving as the groove section 33t. However, the groove section 33t may be not narrowed in this manner, or in other words, the width of the groove section 33t may be uniform along the entire length in the vertical direction.

### ===Layered Product 1 of Sheet-Like Members according to Third Embodiment and Application Example thereof===

A third embodiment is different from the first embodiment in the shape of the embossed depressions 31 when viewed from above. Accordingly, in the following description, the shape of the embossed depressions 31 when viewed from above will be mainly explained using FIG. 9A again.

In a layered body 1 of sheet-like members according to the third embodiment, embossed depressions 31 that are V-shaped or boomerang-shaped when viewed from above are formed. This layered body 1 is used as the surface sheet member 1 in a similar manner as the first embodiment.

The boomerang-shaped embossed depressions 31 are formed at the same positions when viewed from above as the formation positions of the embossed depressions 31 according to the first embodiment. Moreover, the vertex angle of the boomerang shape is an obtuse angle, and the boomerang shape is line symmetric with respect to the bisector of the vertex angle. Thus, the design quality is increased. Herein, the longer of two directions, namely, the direction of the bisector, which is the axis of line symmetry, and the direction perpendicular to the bisector direction, is defined as a longitudinal direction. In this example, the direction perpendicular to the bisector is longer than the direction of the bisector, so that the direction perpendicular to the bisector is the longitudinal direction. The other shorter direction (the direction of the bisector) is referred to as a short length direction.

In the third embodiment, the longitudinal direction of each of the embossed depressions 31 is perpendicular to the MD direction (the vertical direction). Thus, the size of portions protruding from the through holes 12 of the first sheet-like member 11 can be most effectively increased.

For the same reason as described in the first embodiment, it is desirable that both of the length L3 in the longitudinal direction and the length L4 in the short length direction of these embossed depressions 31 also are within the range of 1.5 mm to 10 mm. Therefore, in this application example, the length L3 in the longitudinal direction and the length L4 in the short length direction are 2.29 mm and 1.06 mm, respectively.

### ===Other Embodiments===

In the foregoing, embodiments of the invention were described. However, the invention is not limited to these embodiments, and modifications as described below are possible.

In the foregoing embodiments, the layered body 1 in which the groove sections 13 are formed in the first sheet-like member 11 was described as an example. However, the groove sections 13 does not have to be formed as long as the through holes 12 are formed, or conversely, the groove sections 13 may be formed in both of the first sheet-like member 11 and the second sheet-like member 21.

In the foregoing embodiments, the through holes 12 were formed only in the first sheet-like member 11. However, the invention is not limited thereto. The through holes 12 may be formed in both of the first sheet-like member 11 and the second sheet-like member 21. A layered body 1 with this configuration has excellent fluid permeability.

In the foregoing embodiments, the embossed depressions 31 were formed only in the first sheet-like member 11. However, the invention is not limited thereto. Conversely, the embossed depressions 31 may be formed only in the second sheet-like member 21, or the embossed depressions 31 may be formed in both of the first sheet-like member 11 and the second sheet-like member 21.

In the foregoing embodiments, the layered body 1 having a two-layer structure in which the first sheet-like member 11 and the second sheet-like member 21 are superposed was described as an example of the layered body 1 of sheet-like members. However, the umber of layers is not limited to two, and three or more sheet-like members may be superposed.

In the foregoing embodiments, the longitudinal direction of the through holes 12 in the first sheet-like member 11 was set to the MD direction. However, the invention is not limited thereto. The longitudinal direction may be set to the CD direction or a direction other than the MD direction and the CD direction.

In the foregoing embodiments, an example in which the through holes 12 in the first sheet-like member 11 are elliptical when viewed from above was described. However, as long as the longitudinal direction of the through holes 12 is set to the MD direction, the shape of the through holes 12 when viewed from above is not limited to elliptical shapes. For example, a polygonal shape such as a rectangular shape may also be used.

In the foregoing embodiments, examples in which the embossed depressions 31 are elliptical, boomerang-shaped, or heart-shaped when viewed from above were described. However, as long as the shape of the embossed depressions 31 when viewed from above has a longitudinal direction, the shape is not limited to the above-described shapes. For example, a shape of the letter H or the like may also be used.

In the foregoing embodiments, an application example in which the layered body 1 of sheet-like members is used in a sanitary napkin was described. However, the invention is not limited thereto. For example, the layered body 1 can be used in a disposable diaper and an incontinence absorbent pad, and furthermore, a sanitary product such as a waste cloth for wiping fluid.

In the foregoing embodiments, the first sheet-like member 11 and the second sheet-like member 21 were joined to each other by simply welding these sheet-like members at the embossed depressions 31. However, the joining strength may be reinforced with a hot-melt adhesive that is preliminarily applied, before the embossing, to a face of the second sheet-like member 21 on which the first sheet-like member 11 is placed. Note that in this case, in the layered body 1 after joining, the hot-melt adhesive that has been applied to the second sheet-like member 21 appears through the through holes 12 in the first sheet-like member 11. That is, the hot-melt adhesive is exposed, so that there is a risk that the surface 11a of the first sheet-like member 11 has a sticky touch. In order to prevent this problem, desirably, it is advantageous to use a low tack hot-melt adhesive having a low tackiness. An example of the low tack hot-melt adhesive is a hot-melt adhesive in which an olefinic material is used as the base polymer.

In the foregoing embodiments, nonwoven fabrics were described as examples of the first sheet-like member 11 and the second sheet-like member 21. However, the invention is not limited thereto. For example, woven fabrics or films can also be used.

In the foregoing embodiments, only the first sheet-like member 11 and the second sheet-like member 21 were joined by the embossed depressions 31, but the invention is not limited thereto. For example, the absorbent body 83 located below the second sheet-like member 21 may further be simultaneously joined by the embossed depression 31. That is, the embossed depression 31 may be formed under a state in which the first sheet-like member 11 and the second sheet-like member 21 that are nonwoven fabrics including thermoplastic fiber are placed on the absorbent body 83 so that items from the first sheet-like member 11 to the absorbent body 83 may be integrated. In such a case, even if the absorbent article 81 deforms and is twisted when worn, the first sheet-like member 11 and the second sheet-like member 21 is unlikely to separate from the absorbent body 83. This allows excreted bodily fluid to transfer easily to the absorbent body 83 without remaining in the first sheet-like member 11 or the second sheet-like member 21.

Further, the second sheet-like member 21 may be the absorbent body 83. In this case, the first sheet-like member 11 that is a nonwoven fabric including thermoplastic fiber is in direct contact with the absorbent body 83. Therefore, even when a small amount of bodily fluid is excreted, the fluid can easily tranfer to the absorbent body 83 without remaining in the first sheet-like member 11.

Furthermore, the above-mentioned configurations may be combined along the longitudinal direction of the absorbent article 81. For example, the area that comes into contact with an area near the vaginal opening, the second sheet-like member 21 is a nonwoven fabric including thermoplastic fiber. However, in the areas adjacent to the front and rear of the above area in the longitudinal direction of the absorbent article 81 (hereinafter referred to as the fore-and-aft region), the second sheet-like member 21 can be the absorbent body 83. In this way, the portion of the nonwoven fabric of the second sheet-like member 21 that opposes the vicinity of the vaginal opening from which a large amount of bodily fluid is excreted includes thermoplastic fiber so that it is unlikely that the permeability of bodily fluid is blocked. Therefore, it makes it difficult for the bodily fluid to smear widely at the first sheet-like member 11. Further, the amount of bodily fluid that would flow to the fore-and-aft region along the wearer's body or the like is small. Therefore, the second sheet-like member 21 being the absorbent body 83 allows the bodily fluid to be immediately transferred to the absorbent body 83.

In the foregoing embodiments, although the absorbent body 83 has not described in detail; a commonly used type is adopted. For example, the absorbent body 83 is configured with a so-called air laid nonwoven fabric that is a sheet-like absorbent body material, an absorbent body material and a cover covering the absorbent body material. The absorbent body material consists of absorbent fiber, or consists of absorbent fiber and superabsorbent resin. As an example of the absorbent fiber, there is pulp fiber (pulp that has been pulverized into a fibrous state), and as an example of superabsorbent resin there is super absorbent polymer. Other examples of absorbent fiber may include cellulose such as cotton, regenerated cellulose such as rayon or fibril rayon, semisynthetic cellulose such as acetate or triacetate, fibrous polymer, thermoplastic fiber, and the like. An example of the cover includes thin paper such as tissue paper. The above-mentioned absorbent body 83 can be embossed in order to adjust the thickness or to integrate the absorbent body material and the cover.

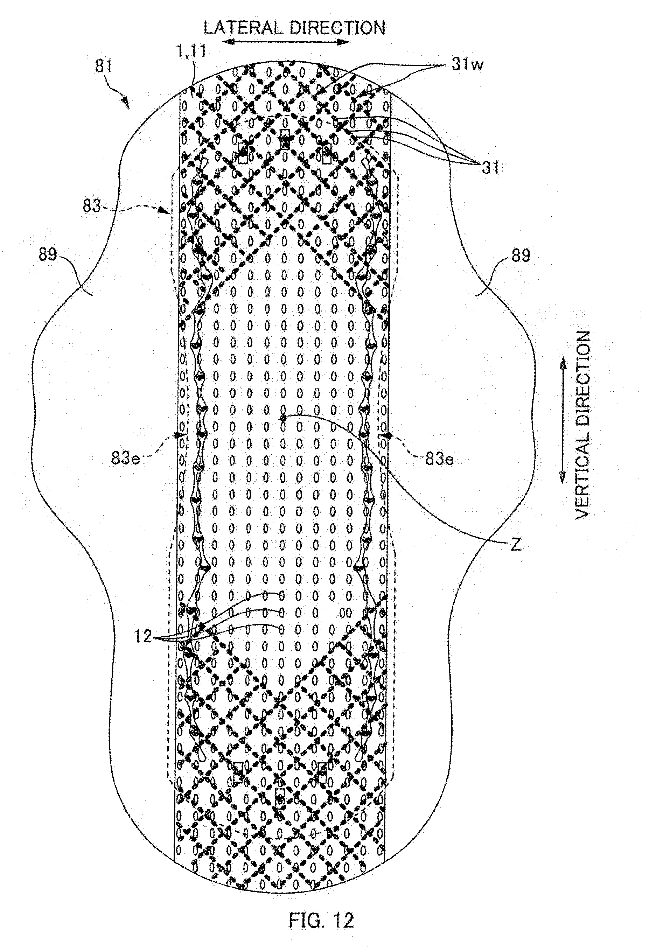

In the foregoing embodiments, a staggered pattern such as that shown in FIG. 1 has been illustrated as an example of a pattern of forming the embossed depression 31. However, other patterns can be adopted. For example, the embossed depressions 31 may be placed in such a manner that, as a whole, the embossed depressions 31 exhibit a grid like pattern (a so-called quilting stitch) (see FIG. 12), by narrowing the spacing between the embossed depressions 31, 31 (that is, the length of a portion where the embossed depressions 31 are not formed) and arranging the embossed depressions in a substantially continuous manner. Alternatively, each line segment 31w that forms the grid-like pattern may be formed with a straight line as shown in FIG. 12, or may be a curved line.

Further, the above-mentioned line segment may be formed in a single continuous groove-like manner, by forming the above-mentioned embossed depressions 31 so that there is no spacing between adjacent embossed depressions 31, that is, by forming the embossed depressions 31 so that the ends of each embossed depression 31 overlap. In this case, the groove itself serves as an "embossed depression"; thereby, if a longitudinal direction of the groove slants with respect to the longitudinal direction of the through hole 12, this configuration is also included within the range of the present invention. Further, in the case of this groove, the longitudinal direction of each embossed depression 31 that forms the groove need not slant with respect to the through hole 12. Furthermore, each embossed depression 31 may be in a shape that does not have a longitudinal direction, such as a perfect circle and a square.

When the embossed depressions 31 are disposed in a substantially continuous manner, it is possible to reduce the entire thickness of the first sheet-like member 11 and the second sheet-like member 21. Therefore, it is possible to provide the absorbent article 1 for use by wearers who prefer a thin absorbent article 1 or for use on days when a small amount of bodily fluid is excreted.

Further, the embossed depression 31 may be formed in a part of the first sheet-like member 11 or may be formed on the entire surface of the first sheet-like member 11.

In the absorbent article 81 according to the second embodiment (see FIG. 10), the low-compression depression 33 has been formed in order to lessen rupture of the first sheet-like member 11 by reducing an abrupt compression deformation due to the embossed depression 31. This is effective particularly in the case where the thickness is comparatively large: a case where the second sheet-like member 21 is the absorbent body 83, or a case where the first sheet-like member 11, the second sheet-like member 21, and the absorbent body 83 are embossed simultaneously. However, the low-compression depression 33 does not have to be provided in the case where the thickness is comparatively small: a case where the first sheet-like member 11 and the second sheet-like member 21 are nonwoven fabrics, or a case where the absorbent body 83 is thin. For example, in FIG. 10, each set of three low-compression depressions 33 disposed at front and rear areas of a product are not required if the absorbent body is thin in these areas.

## Claims

1. A layered body of sheet-like members, comprising:
a first sheet-like member that includes a plurality of through holes having a same longitudinal direction;
a second sheet-like member on which the first sheet-like member is placed; and
a plurality of joining depressions that are formed in at least either one of the first sheet-like member and the second sheet-like member, and that joins the first sheet-like member and the second sheet-like member to each other, a longitudinal direction of each of the joining depressions slanting with respect to the longitudinal direction of the through holes.

2. A layered body of sheet-like members according to claim 1, wherein
the longitudinal direction of each of the joining depressions is perpendicular to the longitudinal direction of the through holes.

3. A layered body of sheet-like members according to claim 1 or 2, wherein
the first sheet-like member has a plurality of groove sections;
a longitudinal direction of each of the groove sections is the same as the longitudinal direction of the through holes;
the groove sections are provided side by side in a direction perpendicular to the longitudinal direction of the groove sections; and
at least one through hole of the plurality of through holes is provided on each of the groove sections.

4. A layered body of sheet-like members according to any of claims 1 to 3, wherein
the first sheet-like member and the second sheet-like member are nonwoven fabrics including thermoplastic fiber;
the first sheet-like member and the second sheet-like member are welded to each other at the joining depressions; and
in the first sheet-like member, an amount of fiber oriented in a direction perpendicular to the longitudinal direction of the through holes is smaller than an amount of fiber oriented in the longitudinal direction of the through holes.

5. A layered body of sheet-like members according to any of claims 1 to 4, wherein
a size of each of the joining depressions in the direction perpendicular to the longitudinal direction of the through holes is larger than a size of the through holes in the direction perpendicular to the longitudinal direction of the through holes by 0.3 to 5 mm.
